# EUROPÄISCHE PATENTANMELDUNG

(11) **EP 4 817 547 A1**
(43) Veröffentlichungstag der Anmeldung: **07.10.2026**
(21) Anmeldenummer: 25168318.1
(22) Anmeldetag: 03.04.2025
(51) Int. Cl.: A61B 34/20, A61B 34/30

(54) **NAVIGATIONSSYSTEM**

(71) Anmelder: B. Braun New Ventures GmbH, 79110 Freiburg im Breisgau (DE)
(72) Erfinder: STEHLE, Simon, 79100 Freiburg (DE); RAMOS, Adrian, 10439 Berlin (DE); PERRUSI, Pedro, 79106 Freiburg (DE)
(74) Vertreter: Winter, Brandl - Partnerschaft mbB

(57) **Zusammenfassung**

Die vorliegende Offenbarung betrifft ein Navigationssystem (2) für einen chirurgischen Eingriff an einem Patienten, mit einem Roboter (4), der eine Roboterbasis (6) und einen zu der Roboterbasis (6) beweglichen Roboterarm (8) mit einem Endeffektor (10) aufweist, einem Robotertracker (18) zum Referenzieren der Position und Orientierung des Roboters (4), einem Patiententracker (20) zum Referenzieren der Position und Orientierung des Patienten, einer Navigationskamera (22), die eingerichtet ist, den Robotertracker (18) nachzuverfolgen und als Robotertracker-Positionsdaten (Trob) bereitzustellen sowie den Patiententracker (20) nachzuverfolgen und als Patiententracker-Positionsdaten (Tpat) bereitzustellen, und einer Steuereinheit (24), die eingerichtet ist, eine Transformationsmatrix (Tref) auf Basis der Robotertracker-Positionsdaten (Trob) und der Patiententracker-Positionsdaten (Tpat) zu bestimmen und eine Veränderung (Tchange) der Transformationsmatrix (Tref) zu überwachen.

## Beschreibung

### Technisches Gebiet

Die vorliegende Offenbarung betrifft ein Navigationssystem für einen chirurgischen Eingriff.

Bei chirurgischen Eingriffen wird oftmals eine Navigation eingesetzt, die den Chirurgen dabei unterstützt, sich basierend auf radiologischen 3D-Aufnahmedaten zu orientieren und den Eingriff gezielt, effektiv und effizient durchzuführen.

Um die Navigation einsetzen zu können, wird zunächst eine Registrierung durchgeführt, bei der eine Transformation zwischen einer zeitaktuellen Position und/oder Orientierung des Patienten und radiologischen 3D-Aufnahmedaten bestimmt wird, um die 3D-Aufnahmedaten oder insbesondere einen rigide am Patienten befestigten und mit den 3D-Aufnahmedaten verknüpften Patiententracker als Referenz während des Eingriffs nutzen und basierend darauf nachverfolgte Instrumente o.ä. navigieren zu können.

Ein Erfolg der Navigation bzw. des navigierten chirurgischen Eingriffs ist jedoch maßgeblich von einer Genauigkeit der Navigation abhängig, da Ungenauigkeiten bei der Navigation gravierende Folgen für die Patientensicherheit haben können. Solche Ungenauigkeiten können insbesondere dadurch entstehen, dass der Patiententracker bewegt wird. Eine Bewegung des Patiententrackers kann beispielsweise durch unbeabsichtigtes Anstoßen des Patiententrackers ausgelöst werden.

Insbesondere ist eine solche Ungenauigkeit besonders kritisch, wenn die Ungenauigkeit nicht bekannt ist oder erkannt wird.

Eine häufig verwendete Methode zur Überprüfung der Navigationsgenauigkeit besteht darin, den Benutzer in bestimmten Zeitabständen aufzufordern, die Navigationsgenauigkeit manuell zu überprüfen. Dies hat jedoch den Nachteil, dass es jedoch zeitintensiv ist und insbesondere bei zu großen Zeitabständen ein Sicherheitsrisiko darstellen kann.

Zudem ist es beispielsweise aus der US 11,857,266 B2 bekannt, einen zusätzlichen Tracker an dem Patienten anzubringen und diesen kontinuierlich zu überwachen. Dies hat jedoch den Nachteil, dass der zusätzliche Tracker angebracht werden muss, dass er ebenfalls unbeabsichtigt verlagert werden kann und dass eine unverdeckte, direkte Sichtlinie zur Navigationskamera benötigt wird, um die kontinuierliche Überwachung zu ermöglichen.

Aus der EP 2 967 297 B1 ist ein System zur dynamischen Validierung einer chirurgischen Navigation bekannt, bei der eine nachverfolgte Kamera auf einen Operationssitus bzw. eine Patientenanatomie ausgerichtet ist und Augmented Reality (AR) eingesetzt wird. Dabei werden eine virtuelle Darstellung und eine zeitaktuelle Aufnahme von verwendeten Instrumenten oder der Patientenanatomie überlagert, um eine potentielle Diskrepanz feststellen zu können. Im Fall einer Abweichung zwischen der virtuellen Darstellung und der zeitaktuellen Aufnahme ist es klar, dass die Genauigkeit der chirurgischen Navigation reduziert ist. Nachteilig daran ist jedoch, dass eine nachverfolgte Videokamera erforderlich ist, die auf das Operationsgeschehen ausgerichtet ist. Insbesondere bei stereotaktischen Eingriffen, bei denen im Gegensatz zu mikrochirurgischen Eingriffen keine Kamera ohnehin auf das Operationsgeschehen ausgerichtet sein muss, ist eine solche Validierung nur mit zusätzlichem Aufwand einsetzbar.

Weiter ist aus der US 11,191,595 B2 und der US 12,023,107 B2 eine Lösung bekannt, bei der eine zweite Registrierung mit einem 3D-Scanner durchgeführt wird, wobei der 3D-Scanner einen Oberflächenscan durchführt und die Punktwolke mit der Punktwolke der radiologischen 3D-Aufnahmedaten abgleicht. Da der Patient und insbesondere ein merkmalsreiches Gesicht des Patienten während des Eingriffs abgedeckt bleiben muss, hat diese Lösung jedoch den Nachteil, dass die zweite Registrierung ggf. nicht genau genug ist. Zudem kann nicht auf eine Bewegung der Navigationskamera reagiert werden.

Es ist also die Aufgabe der vorliegenden Offenbarung, die Nachteile aus dem Stand der Technik zu vermeiden oder wenigstens zu verringern. Insbesondere soll ein Navigationssystem bereitgestellt werden, das eine Navigation mit besonders hoher Genauigkeit ermöglicht, die vor allem robust gegenüber Fehlern durch unbeabsichtigtes Bewegen eines Patiententrackers ist.

Die Aufgabe der vorliegenden Offenbarung wird durch ein Navigationssystem mit den Merkmalen des Patentanspruchs 1 gelöst. Vorteilhafte Weiterbildungen sind Gegenstand der abhängigen Ansprüche.

Demnach betrifft die vorliegende Offenbarung ein Navigationssystem für einen chirurgischen, insbesondere neurochirurgischen, Eingriff an einem Patienten. Das Navigationssystem weist einen Roboter auf. Der Roboter weist eine Roboterbasis und einen zu der Roboterbasis beweglichen Roboterarm mit einem Endeffektor auf. Das Navigationssystem weist einen Robotertracker zum Referenzieren der Position und Orientierung des Roboters auf. Der Robotertracker ist rigide an dem Roboter befestigt. Das Navigationssystem weist einen Patiententracker zum Referenzieren der Position und Orientierung des Patienten auf. Der Patiententracker ist eingerichtet, rigide an dem Patienten befestigt zu werden. Das Navigationssystem weist eine Navigationskamera auf. Die Navigationskamera ist eingerichtet, den Robotertracker (kontinuierlich) nachzuverfolgen und als Robotertracker-Positionsdaten bereitzustellen. Die Navigationskamera ist eingerichtet, den Patiententracker (kontinuierlich) nachzuverfolgen und als Patiententracker-Positionsdaten bereitzustellen. Das Navigationssystem weist eine Steuereinheit auf. Die Steuereinheit ist eingerichtet, eine Transformationsmatrix auf Basis der Robotertracker-Positionsdaten und der Patiententracker-Positionsdaten zu bestimmen. Die Steuereinheit ist eingerichtet, eine (zeitliche) Veränderung der Transformationsmatrix (kontinuierlich) zu überwachen. Die Überwachung dient insbesondere dazu, um eine Relativbewegung zwischen dem Patienten und dem Patiententracker zu erkennen.

Der Kern der vorliegenden Offenbarung besteht demzufolge darin, dass eine Relativposition und Relativorientierung zwischen dem Patiententracker und dem Roboter oder dem Robotertracker als eine Referenz verwendet wird und diese Referenz während des Eingriffs überwacht wird. Dabei verändert sich die Relativposition und/oder Relativorientierung in dem Fall, in dem sich zumindest einer aus Patient, Patiententracker, Roboter und Robotertracker relativ zu einem der anderen aus Patient, Patiententracker, Roboter und Robotertracker bewegt. Wenn sich die relative Transformation verändert, können anhand der Veränderung vorzugsweise Rückschlüsse darüber gezogen werden, ob sich der Patiententracker in unbeabsichtigter Weise (d.h. alleine/relativ zu dem Patienten) oder in beabsichtigter Weise (d.h. gemeinsam/zusammen mit dem Patienten) oder ob sich der Roboter in beabsichtigter oder unbeabsichtigter Weise (relativ zu dem Patienten) bewegt hat.

Die vorliegende Offenbarung hat den Vorteil, dass durch das kontinuierliche Überwachen eine potentielle Ungenauigkeit in der Navigation durch das Navigationssystem besonders frühzeitig, einfach und zuverlässig erkannt werden kann, so dass die Patientensicherheit erhöht wird.

Mit anderen Worten betrifft die vorliegende Offenbarung ein Navigationssystem, bei dem mittels einer Navigationskamera ein Roboter (über einen Robotertracker) sowie ein Patient (über einen Patiententracker) nachverfolgt sind und bei dem mittels einer Steuereinheit eine Relativposition und eine Relativorientierung zwischen dem Robotertracker und dem Patiententracker als Referenz zeitlich/kontinuierlich überprüft wird und eine Änderung der Relativposition und der Relativorientierung als Indikator für eine potentielle Veränderung einer Navigationsgenauigkeit verwendet wird.

Wie oben beschrieben ist der Robotertracker rigide mit dem Roboter verbunden. Das heißt, dass der Robotertracker so an dem Roboter angebracht ist, dass (möglichst) keine Relativbewegung (zwischen dem Roboter, d.h. zumindest des Roboterbestandteils, an dem der Robotertracker angebunden ist, und dem Robotertracker) auftritt.

Wie oben beschrieben ist der Patiententracker rigide mit dem Patienten verbunden. Das heißt, dass der Patiententracker so an dem Patienten angebracht ist, dass (möglichst) keine Relativbewegung (zwischen dem Patienten und dem Patiententracker) auftritt. Insbesondere kann der Patiententracker mit einem Körperteil des Patienten, an welchem der Eingriff stattfinden soll, verbunden sein. Der Patiententracker kann direkt oder indirekt an dem Patienten angebracht sein. Das heißt, dass der Patiententracker auch an einer rigide an dem Patienten befestigten Vorrichtung angebracht sein kann. Beispielsweise kann der Patiententracker im Fall eines neurochirurgischen Eingriffs an einer Kopfklemme angebracht sein.

Wie oben beschrieben werden der Robotertracker und der Patiententracker durch die Navigationskamera nachverfolgt. Dazu sind der Robotertracker und der Patiententracker innerhalb eines Sichtfelds der Navigationskamera angeordnet. Die Navigationskamera kann beweglich sein. Die Navigationskamera kann für eine Nachverfolgung (Tracking) im IR-Bereich und/oder im sichtbaren Spektrum eingerichtet sein.

Gemäß einer bevorzugten Ausführungsform kann die Steuereinheit eingerichtet sein, die Veränderung der Transformationsmatrix mit einem vorbestimmten Schwellenwert zu vergleichen.

Gemäß einer bevorzugten Ausführungsform kann die Steuereinheit eingerichtet sein, bei Überschreiten des vorbestimmten Schwellenwerts einen Hinweis für einen Benutzer auszugeben. Das heißt, dass die Steuereinheit eingerichtet ist, den Benutzer zunächst einmal auf die Veränderung der Transformationsmatrix aufmerksam zu machen. Dies hat den Vorteil, dass der Benutzer selbst eine informierte Entscheidung darüber treffen kann, ob und/oder in welcher Weise sich die Veränderung der Position des Robotertrackers und/oder Patiententracker ggf. auf geplante Schritte auswirkt oder auswirken könnte. Es wird also ein Bewusstsein des Benutzers über die Positions-/Orientierungsänderung geschaffen.

Gemäß einer bevorzugten Weiterbildung kann der Hinweis graphisch und/oder akustisch sein. So kann ein geeignetes Mittel gewählt werden, um den Benutzer auf die erfasste Relativbewegung hinzuweisen.

Gemäß einer bevorzugten Weiterbildung kann der Hinweis kann in Abhängigkeit der Veränderung der Transformationsmatrix variieren. Beispielsweise kann der Hinweis bei einer bestimmten (z.B. bei einer größeren oder z.B. bei einer auf eine beabsichtigte Relativbewegung hindeutenden) Veränderung stärker hervorgehoben sein als bei einer anderen (z.B. bei einer kleineren oder z.B. bei einer auf eine unbeabsichtigte Relativbewegung hindeutenden) Veränderung. So kann ein geeignetes Mittel gewählt werden, um den Benutzer ausreichend aufmerksam zu machen und gleichzeitig nicht allzu sehr von seiner aktuellen Tätigkeit abzulenken.

Gemäß einer bevorzugten Ausführungsform kann die Steuereinheit eingerichtet sein, bei Überschreiten des vorbestimmten Schwellenwerts einen Benutzer aufzufordern, eine Navigationsgenauigkeit manuell zu überprüfen. Die manuelle Überprüfung kann insbesondere dadurch erfolgen, dass der Benutzer einen nachverfolgten Pointer zu vorbestimmen Landmarken des Patienten bewegt und/oder sich eine virtuelle Darstellung und eine zeitaktuelle Aufnahme eines nachverfolgten Instruments als Überlagerungsdarstellung mittels Augmented Reality darstellen lässt. Vorzugsweise kann die Steuereinheit eingerichtet sein, den Benutzer aufzufordern, die Navigationsgenauigkeit nach der manuellen Überprüfung zu bestätigen.

Gemäß einer bevorzugten Ausführungsform kann der Robotertracker rigide an dem Roboterarm befestigt sein. Insbesondere kann der Robotertracker rigide an dem Endeffektor des Roboterarms, d.h. endständig, angebunden sein. Der Endeffektor bildet (von der Roboterbasis aus gesehen) das letzte bewegliche Robotersegment des Roboterarms. Innerhalb eines Robotersegments ist kein Freiheitsgrad realisiert.

Gemäß einer bevorzugten Ausführungsform kann die Steuereinheit eingerichtet sein, in einem roboterstatischen Fall eine erste Transformationsmatrix als die Transformationsmatrix zu verwenden, wobei die erste Transformationsmatrix eine relative Position und eine relative Orientierung zwischen dem Robotertracker (und damit dem Roboterarm, insbesondere dem Endeffektor) und dem Patiententracker (und damit dem Patienten) angibt. Unter dem roboterstatischen Fall wird eine Situation verstanden, bei der sich der Roboterarm während der Überwachung nicht bewegt hat. Dies hat den Vorteil, dass die starre Transformation zwischen dem Endeffektor und dem Patiententracker zur Überwachung verwendet werden kann.

Gemäß einer bevorzugten Ausführungsform kann die Steuereinheit eingerichtet sein, Roboterbasis-Positionsdaten auf Basis der Robotertracker-Positionsdaten und einer Roboterkinematik des Roboters zu bestimmen. Das heißt, dass eine Position der Roboterbasis rückgerechnet werden kann.

Gemäß einer bevorzugten Ausführungsform kann die Steuereinheit eingerichtet sein, in einem roboterdynamischen Fall eine zweite Transformationsmatrix als die Transformationsmatrix zu verwenden, wobei die zweite Transformationsmatrix eine relative Position und eine relative Orientierung zwischen der Roboterbasis und dem Patiententracker angibt. Unter dem roboterdynamischen Fall wird eine Situation verstanden, bei der sich der Roboterarm während der Überwachung bewegt hat. Dies hat den Vorteil, dass die starre Transformation zwischen der Roboterbasis und dem Patiententracker zur Überwachung verwendet werden kann.

Mit anderen Worten wird für die kontinuierliche Überwachung der Genauigkeit zwischen dem statischen Fall, bei dem sich der Roboterarm bzw. der Endeffektor nicht bewegt, und dem dynamischen Fall, bei dem sich der Roboterarm bzw. der Endeffektor bewegt, unterschieden werden. Im statischen Fall wird die Transformation zwischen dem Patiententracker und des am Endeffektor angebrachten Robotertrackers als fest angenommen und kontinuierlich überwacht. Wenn sich der Roboterarm bzw. der Endeffektor bewegt, ist der Roboterarm bzw. der Endeffektor nicht mehr statisch in Bezug auf den Patiententracker, aber die Roboterbasis ist statisch. Daher wird während der Roboterbewegung die Transformation zwischen der Basis und dem Patiententracker überwacht, wozu die Roboterkinematik verwendet wird, um die Position der Basis relativ zum Roboterarm bzw. Endeffektor zu berechnen. Ändert sich die Transformation (zwischen dem Patiententracker und dem Roboterarm bzw. Robotertracker (im statischen Fall) oder der Roboterbasis (im dynamischen Fall) über den vorbestimmten Schwellenwert hinaus, kann davon ausgegangen werden, dass der Patiententracker bewegt wurde oder jemand gegen ihn gestoßen ist, worüber der Benutzer durch den Hinweis informiert wird und dann die Möglichkeit hat, Korrekturen anzuwenden.

Gemäß einer bevorzugten Ausführungsform kann die Steuereinheit eingerichtet sein, in dem roboterstatischen Fall einen ersten vorbestimmten Schwellenwert und in dem roboterdynamischen Fall einen zweiten vorbestimmten Schwellenwert als den Schwellenwert zu verwenden. Dabei können der erste vorbestimmte Schwellenwert und der zweite vorbestimmte Schwellenwert gleich sein oder unterschiedlich zueinander sein. Dies hat den Vorteil, dass der Hinweis für den Benutzer situationsangepasst generiert wird und somit besonders informativ ist.

Gemäß einer bevorzugten Ausführungsform kann das Navigationssystem radiologische 3D-Aufnahmedaten (3D, dreidimensionale) des Patienten aufweisen. Die 3D-Aufnahmedaten können beispielsweise mittels CT, MRT, Röntgen oder Ultraschall, vorzugsweise präoperativ und/oder intraoperativ, gewonnen werden und zeigen innere Strukturen bzw. inneres Gewebe des Patienten. Die radiologischen 3D-Aufnahmedaten können als ein 3D-Modell oder in Form von 2D-Schnittbildern, die ein 3D-Modell bilden können, vorliegen.

Gemäß einer bevorzugten Ausführungsform kann das Navigationssystem eine Aufnahmeeinheit aufweisen. Die Aufnahmeeinheit ist eingerichtet, eine zeitaktuelle Aufnahme des Patienten zu erstellen. Die zeitaktuelle Aufnahme kann eine bildbasierte Aufnahme des Patienten sein. In diesem Fall ist die Aufnahmeeinheit eine Kamera. Die zeitaktuelle Aufnahme kann eine pointerbasierte Aufnahme sein. In diesem Fall ist die Aufnahmeeinheit ein Pointer.

Gemäß einer bevorzugten Ausführungsform kann die Steuereinheit eingerichtet sein, eine (initiale) Registrierung der radiologische 3D-Aufnahmedaten zu dem Patienten, insbesondere anhand der zeitaktuellen Aufnahme des Patienten, durchzuführen und die radiologischen 3D-Aufnahmedaten fest mit dem (rigide an dem Patienten befestigten) Patiententracker zu verknüpfen. Dabei wird eine statische bzw. konstante Transformation zwischen den radiologischen 3D-Aufnahmedaten und dem Patienten in seiner zeitaktuellen Position und Orientierung bestimmt und durch eine Position und eine Orientierung des Patiententrackers referenziert. Dies hat den Vorteil, dass das Nachverfolgen des Patiententrackers ein Nachverfolgen einer Bewegung des Patienten ermöglicht. Die Registrierung kann vorzugsweise (nur) initial, insbesondere präoperativ oder intraoperativ zu Beginn des Eingriffs, durchgeführt werden oder wiederholt, insbesondere intraoperativ während des Eingriffs, durchgeführt werden.

Gemäß einer bevorzugten Ausführungsform kann die Steuereinheit eingerichtet sein, die (initiale) Registrierung auf Basis der Veränderung der Transformationsmatrix zu korrigieren. Das heißt, dass die Steuereinheit eingerichtet ist, auf Basis der Veränderung der Transformationsmatrix zu bestimmen, ob und/oder in welcher Weise die Korrektur der Registrierung erforderlich ist. Insbesondere kann die Steuereinheit eingerichtet sein, anhand von hinterlegten Steuerungsanweisungen, wie etwa die Veränderung der Transformationsmatrix über einen bestimmten Schwellenwert und/oder über eine bestimmte Zeitdauer, eine Aufforderung über eine Korrekturmöglichkeit an den Benutzer auszugeben.

Gemäß einer bevorzugten Weiterbildung kann die Steuereinheit eingerichtet sein, die Registrierung in Reaktion auf eine Benutzereingabe zu korrigieren. Das heißt, dass die Registrierung nicht automatisch angepasst wird, sondern der Benutzer über die Anpassung/Korrektur der Registrierung entscheidet oder diese freigibt/bestätigt. So bleiben die Entscheidungsgewalt und die Verantwortung beim Benutzer, so dass ggf. fehlerhafte Rückschlüsse der Steuereinheit vermieden werden können. Zudem kann der Benutzer so über die Korrektur der Registrierung aufmerksam gemacht werden.

Alternativ kann die Steuereinheit eingerichtet sein, die Registrierung automatisch zu korrigieren. Das heißt, dass die Steuereinheit anhand von hinterlegten Steuerungsanweisungen bestimmt, dass die Anpassung/Korrektur der Registrierung erforderlich ist und diese automatisch veranlasst. So können ggf. fehlerhafte Einschätzungen des Benutzers vermieden werden.

Gemäß einer bevorzugten Weiterbildung kann die Steuereinheit eingerichtet sein, die Registrierung um einen Korrekturfaktor in Form einer Korrekturmatrix zu korrigieren.

Gemäß der bevorzugten Weiterbildung kann die Steuereinheit eingerichtet sein, in dem roboterstatischen Fall die Veränderung der Transformationsmatrix als die Korrekturmatrix zu verwenden. Somit kann die Registrierung in diesem Fall einfach korrigiert werden, da die Bewegung des Patiententrackers zu dem Robotertracker der Bewegung des Patiententrackers zu dem Patienten entspricht. Mit anderen Worten ist die (initale) Registrierung in sich gesehen noch gültig, da die Transformation zwischen der neuen Position und Orientierung des Patiententrackers und der alten Position und Orientierung des Patiententrackers bekannt ist sowie die Transformation zwischen der alten Position und Orientierung des Patiententrackers und der Position und Orientierung des Patienten (unverändert) bekannt ist. Dies hat den Vorteil, dass in diesem Fall keine erneute Registrierung erforderlich.

Gemäß der bevorzugten Weiterbildung kann die Steuereinheit eingerichtet sein, in dem roboterdynamischen Fall den Roboter in eine vorbestimmte Position zu verfahren und die Veränderung der Transformationsmatrix als die Korrekturmatrix zu verwenden. Das heißt, dass die Steuereinheit eingerichtet ist, den Roboter zuvor in eine vorbestimmte Position zu verfahren (und erst anschließend die Registrierung um die Korrekturmatrix zu korrigieren). So kann auf besonders einfache Weise die Korrektur vorgenommen werden.

Gemäß der bevorzugten Weiterbildung kann die Steuereinheit eingerichtet sein, den Roboterarm in Reaktion auf eine Benutzereingabe in die vorbestimmte Position zu verfahren. Das heißt, dass der Roboter nicht automatisch verfahren wird, sondern der Benutzer entscheidet oder das Verfahren freigibt/bestätigt.

Gemäß einer alternativen Weiterbildung kann die Steuereinheit eingerichtet sein, in dem roboterdynamischen Fall die Veränderung der Transformationsmatrix (direkt, d.h. ohne Verfahren in eine vorbestimmte Position) als die Korrekturmatrix zu verwenden. In diesem Fall sollte der Roboter besonders genau kalibriert sein.

Gemäß einer bevorzugten Ausführungsform kann die Steuereinheit eingerichtet sein, nach Korrektur der Registrierung einen Benutzer aufzufordern, eine Navigationsgenauigkeit manuell zu überprüfen. Die manuelle Überprüfung kann insbesondere dadurch erfolgen, dass der Benutzer einen nachverfolgten Pointer zu vorbestimmen Landmarken des Patienten bewegt und/oder sich eine virtuelle Darstellung und eine zeitaktuelle Aufnahme eines nachverfolgten Instruments als Überlagerungsdarstellung mittels Augmented Reality darstellen lässt. Vorzugsweise kann die Steuereinheit eingerichtet sein, den Benutzer aufzufordern, die Navigationsgenauigkeit nach der manuellen Überprüfung zu bestätigen.

Gemäß einer bevorzugten Ausführungsform kann die Steuereinheit eingerichtet sein, auf Basis der Veränderung der Transformationsmatrix einen Benutzer aufzufordern, die Registrierung zu wiederholen. Das heißt, dass die Steuereinheit eingerichtet ist, auf Basis der Veränderung der Transformationsmatrix zu bestimmen, ob die Wiederholung der Registrierung erforderlich ist. Insbesondere kann die Steuereinheit eingerichtet sein, anhand von hinterlegten Steuerungsanweisungen, wie etwa die Veränderung der Transformationsmatrix über einen bestimmten Schwellenwert und/oder über eine bestimmte Zeitdauer, eine Aufforderung zur Wiederholung der Registrierung an den Benutzer auszugeben, insbesondere, wenn die Registrierung (beispielsweise aufgrund einer unklaren Veränderung der Transformationsmatrix) nicht korrigierbar ist.

Gemäß einer bevorzugten Ausführungsform kann das Navigationssystem eine Patienten-Bewegungserfassungseinheit aufweisen. Die Patienten-Bewegungserfassungseinheit kann eingerichtet sein, eine (beabsichtigte) Bewegung des Patienten zu erfassen. Die Erfassung der Bewegung des Patienten hat den Vorteil, dass bestimmt werden kann, ob sich bei der Bewegung des Patiententrackers (relativ zu dem Roboter) der Patiententracker mit dem Patienten mitbewegt hat oder relativ zu dem Patienten bewegt hat.

Gemäß einer bevorzugten Weiterbildung kann die Patienten-Bewegungserfassungseinheit eine Verarbeitungseinheit aufweisen, die eingerichtet ist, eine Veränderung der Patiententracker-Positionsdaten, insbesondere eine Veränderungsrichtung und/oder Veränderungsgeschwindigkeit, zu verarbeiten und basierend darauf die Bewegung des Patienten zu bestimmen. Dies hat den Vorteil, dass kein zusätzlicher Sensor erforderlich ist, sondern allein eine Analyse der Patiententracker-Positionsdaten eine ausreichende Aussagekraft bietet.

Insbesondere kann die Verarbeitungseinheit eingerichtet sein, anhand von hinterlegten Steuerungsanweisungen die Bewegung des Patienten zu bestimmen. Die Steuerungsanweisungen können vorzugsweise die Veränderung der Patiententracker-Positionsdaten entlang einer bestimmten Veränderungsrichtung betreffen. Beispielsweise können die Steuerungsanweisungen eine Veränderungsrichtung (nur) in Vertikalrichtung und/oder (nur) in Horizontalrichtung betreffen. Die Steuerungsanweisungen können vorzugsweise die Veränderung der Patiententracker-Positionsdaten mit einer bestimmten Veränderungsgeschwindigkeit betreffen. Beispielsweise können die Steuerungsanweisungen eine Veränderungsgeschwindigkeit größer als ein vorbestimmter Geschwindigkeitswert und/oder mit einem konstanten Geschwindigkeitswert über eine vorbestimmte Zeitdauer und/oder mit einem bestimmten Geschwindigkeitsprofil betreffen.

Gemäß einer bevorzugten Weiterbildung kann die Patienten-Bewegungserfassungseinheit eine Verarbeitungseinheit aufweisen, die eingerichtet ist, einen Steuerungsinput für einen Operationstisch mit einer Liegefläche für den Patienten zu verarbeiten und basierend darauf die Bewegung des Patienten zu bestimmen. Das heißt, dass eine Operationstisch-Steuerungsanweisung zum Ausführen einer bestimmten Bewegung verwendet wird. Dies hat den Vorteil, dass die Bewegung des Patienten besonders zuverlässig und mit verhältnismäßig geringem Aufwand ermittelt werden kann.

Gemäß einer bevorzugten Weiterbildung kann die Patienten-Bewegungserfassungseinheit einen Beschleunigungssensor aufweisen, der eingerichtet ist, rigide mit dem Patienten gekoppelt zu werden. Insbesondere kann der Beschleunigungssensor eingerichtet sein, an einem Operationstisch mit einer Liegefläche für den Patienten angebracht zu sein. Das heißt, dass ein zusätzlicher Sensor verwendet wird, um die Bewegung des Patienten zu erfassen. Dies hat den Vorteil, dass die Bewegung des Patienten besonders zuverlässig ermittelt werden kann.

Gemäß der bevorzugten Ausführungsform kann die Steuereinheit eingerichtet sein, die Transformationsmatrix auf Basis eines Vergleichs der Bewegung des Patienten und der Veränderung der Transformationsmatrix zu korrigieren. Das heißt, dass die Steuereinheit eingerichtet ist, auf Basis der Veränderung der Transformationsmatrix zu bestimmen, ob und/oder in welcher Weise die Korrektur der Transformationsmatrix erforderlich ist. Insbesondere kann die Steuereinheit eingerichtet sein, anhand von hinterlegten Steuerungsanweisungen, wie etwa die Veränderung der Transformationsmatrix über einen bestimmten Schwellenwert und/oder über eine bestimmte Zeitdauer, eine Aufforderung über eine Korrekturmöglichkeit an den Benutzer auszugeben.

Gemäß einer bevorzugten Weiterbildung kann die Steuereinheit eingerichtet sein, die Transformationsmatrix in Reaktion auf eine Benutzereingabe zu korrigieren. Das heißt, dass die Transformationsmatrix nicht automatisch angepasst wird, sondern der Benutzer über die Anpassung/Korrektur der Registrierung entscheidet oder diese freigibt/bestätigt. So bleiben die Entscheidungsgewalt und die Verantwortung beim Benutzer, so dass ggf. fehlerhafte Rückschlüsse der Steuereinheit vermieden werden können. Zudem kann der Benutzer so über die Korrektur der Transformationsmatrix aufmerksam gemacht werden.

Alternativ kann die Steuereinheit eingerichtet sein, die Transformationsmatrix automatisch zu korrigieren. Das heißt, dass die Steuereinheit anhand von hinterlegten Steuerungsanweisungen erkennt, dass die Anpassung/Korrektur der Transformationsmatrix erforderlich ist und diese automatisch veranlasst. So können ggf. fehlerhafte Einschätzungen des Benutzers vermieden werden.

Gemäß einer bevorzugten Weiterbildung kann die Steuereinheit eingerichtet sein, die Transformationsmatrix um einen Korrekturfaktor in Form einer Matrix, die der Veränderung der Transformationsmatrix entspricht, zu korrigieren. Das heißt, dass die Transformationsmatrix in diesem Fall einfach korrigiert werden kann, da zwischen dem Patiententracker und dem Patienten keine Relativbewegung erfolgt ist.

Gemäß einer bevorzugten Ausführungsform kann das Navigationssystem ein digitales Operationsmikroskop aufweisen, das an dem Endeffektor des Roboterarms angebunden ist oder den Endeffektor bildet. Das Operationsmikroskop ist eingerichtet, eine mikroskopische Aufnahme des Patienten zu erstellen. Digital heißt, dass das Operationsmikroskop eingerichtet ist, eine digitale Aufnahme zu erstellen und (insbesondere zur Wiedergabe auf einer Anzeigevorrichtung) bereitzustellen. Das Operationsmikroskop kann stereoskopisch sein. Das heißt, dass das Operationsmikroskop eine (räumliche) Tiefenwahrnehmung hat, bei der eine Tiefeninformation insbesondere aus der Disparität zwischen zwei Aufnahmen, die aus räumlich zueinander versetzten Positionen erstellten sind, generiert wird.

Gemäß einer bevorzugten Ausführungsform kann das Navigationssystem eine Roboterbasis-Bewegungserfassungseinheit, vorzugsweise in Form eines Sensors in einer Bremse der Roboterbasis, aufweisen. Die Roboterbasis-Bewegungserfassungseinheit kann eingerichtet sein, eine (unbeabsichtigte) Bewegung der Roboterbasis zu erfassen. So kann erkannt werden, dass die vorbestimmte Position zur Korrektur in dem roboterdynamischen Fall nicht mehr gültig ist. Vorzugsweise kann die Steuereinheit eingerichtet sein, den Benutzer über die Bewegung der Roboterbasis zu informieren und/oder den Benutzer aufzufordern, die vorbestimmte Position zu aktualisieren. Das Aktualisieren der vorbestimmten Position kann insbesondere dadurch erfolgen, dass der Roboter so bewegt wird, dass sowohl der Robotertracker als auch der Patiententracker in dem Sichtfeld der Navigationskamera liegen.

Gemäß einer bevorzugten Ausführungsform kann das Navigationssystem ein vorzugsweise am Endeffektor angebrachtes Benutzereingabemittel, insbesondere in Form eines Touchscreens, aufweisen. Über das Benutzereingabemittel können die oben beschriebenen Benutzereingaben erfolgen.

Gemäß einer bevorzugten Ausführungsform kann das Navigationssystem eine Benutzereingabemittel-Bewegungserfassungseinheit, vorzugsweise in Form eines Sensors zur Drehmomentmessung in Gelenken des Roboterarms, aufweisen. Die Benutzereingabemittel-Bewegungserfassungseinheit kann eingerichtet sein, eine Bewegung des Benutzereingabemittels zu erfassen. Dies hat den Vorteil, dass eine steifigkeitsbedingte (kurzfristige) Verlagerung des Endeffektors, die durch eine Interaktion des Benutzers mit dem Benutzereingabemittel hervorgerufen wird, bei der Überwachung der Veränderung der Transformationsmatrix herausgefiltert bzw. unberücksichtigt bleiben kann. Dies hat den Vorteil, dass es nicht zu fälschlichen Warnungen des Benutzers kommt.

Gemäß einer bevorzugten Ausführungsform kann die Steuereinheit eingerichtet sein, in einem Fall, in dem der Robotertracker nicht innerhalb des Sichtfelds der Navigationskamera liegt, insbesondere in Folge einer Benutzereingabe, den Roboter in eine vorbestimmte Position, beispielsweise eine zuletzt gespeicherte Position, zu verfahren und diese mit der zuvor überwachten Position zu vergleichen. Wenn die relative Position des Endeffektors zum Patiententracker über einen Schwellenwert hinaus abweicht, wird der Benutzer aufgefordert, die Navigationsgenauigkeit zu testen und ggf. die Korrektur anzuwenden oder die Abweichung zu ignorieren.

Gemäß einer bevorzugten Ausführungsform kann das Navigationssystem ein Instrument aufweisen, wobei die Navigationskamera eingerichtet ist, das Instrument nachzuverfolgen.

Zusammenfassend betrifft die vorliegende Offenbarung ein System, das die Genauigkeit der Navigation im Einsatz kontinuierlich überwachen und korrigieren kann. Die relative starre Transformation zwischen dem Patiententracker in Bezug auf den Roboter wird als Referenz verwendet und während des gesamten Betriebs überwacht. Wenn sich diese Transformation über einen bestimmten Schwellenwert hinaus verändert, ist dies ein deutlicher Hinweis darauf, dass sich der Patiententracker bewegt hat, und der Benutzer wird vor die Wahl gestellt, diese Abweichung zu akzeptieren oder zu korrigieren. Wenn sich der Patient zusammen mit dem Patienten-Tracker bewegt hat, z. B. aufgrund einer Bettbewegung, sollte die Änderung akzeptiert werden, da sie keine Auswirkungen auf die Navigationsgenauigkeit hat, und es wird eine neue Referenztransformation festgelegt. Wenn sich der Patiententracker ohne die Absicht, den Patienten zu bewegen, bewegt hat, wird die Navigationsgenauigkeit beeinträchtigt, und der Benutzer kann wählen, ob er eine Korrekturtransformation anwenden will, um die festgestellte Navigationsabweichung zu korrigieren.

Kurzbeschreibung der Figuren
Fig. 1 zeigt eine schematische Darstellung eines Navigationssystems gemäß der vorliegenden Offenbarung und eine Navigationsgenauigkeitsüberwachung in einem roboterstatischen Fall.
Fig. 2 zeigt eine schematische Darstellung des Navigationssystems gemäß der vorliegenden Offenbarung und eine Navigationsgenauigkeitsüberwachung in einem roboterdynamischen Fall.
Fig. 3 zeigt eine schematische Darstellung des Navigationssystems gemäß der vorliegenden Offenbarung und eine Korrektur einer Registrierung.

### Beschreibung einer bevorzugten Ausführungsform

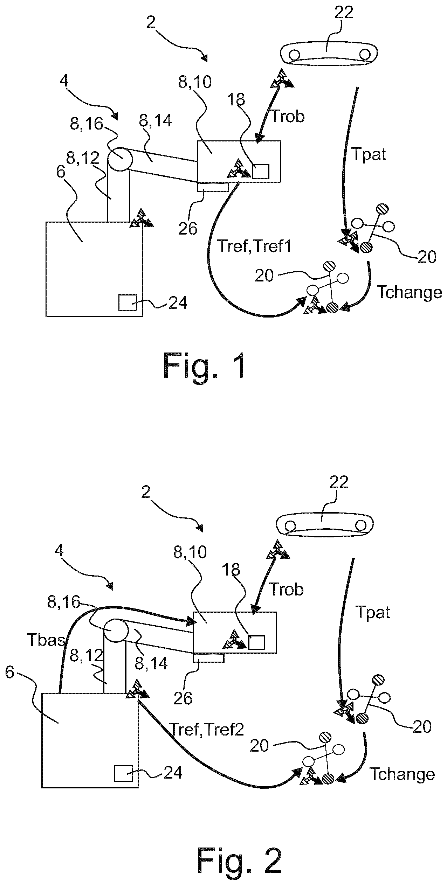

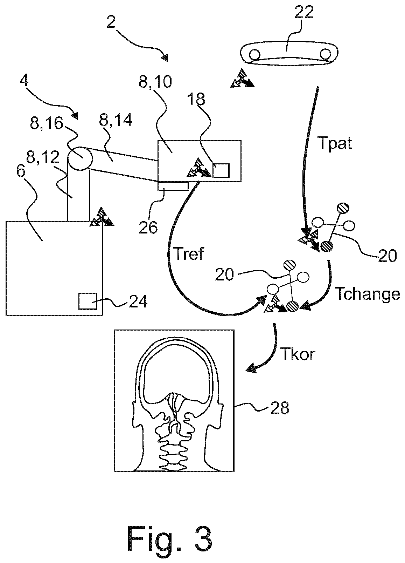

In Fign. 1 bis 3 ist ein Navigationssystem 2 gemäß der vorliegenden Offenbarung schematisch dargestellt. Das Navigationssystem 2 dient für einen chirurgischen, insbesondere neurochirurgischen, Eingriff an einem Patienten.

Das Navigationssystem 2 weist einen Roboter 4 auf. Der Roboter 4 weist eine Roboterbasis 6 und einen zu der Roboterbasis 6 beweglichen Roboterarm 8 mit einem Endeffektor 10 auf. In der dargestellten Ausführungsform weist der Roboterarm 8 zwei zueinander bewegliche Roboterarmsegmente 12, 14 auf, die über ein Gelenk 16 miteinander verbunden sind. Ein erstes Roboterarmsegment 12 ist direkt mit der Roboterbasis 6 verbunden und der Endeffektor 10 ist direkt mit dem zweiten Roboterarmsegment 14 verbunden. Der Roboterarm 8 kann aber auch mehr als zwei Roboterarmsegmente haben.

Vorzugsweise ist ein digitales Operationsmikroskop an dem Endeffektor 10 befestigt oder bildet den Endeffektor 10.

Das Navigationssystem 2 weist einen Robotertracker 18 zum Referenzieren der Position und Orientierung des Roboters 4 auf. Der Robotertracker 18 ist rigide an dem Roboter 4 befestigt. Vorzugsweise ist der Robotertracker 18 rigide an dem Roboterarm 8 befestigt. Insbesondere ist der Robotertracker 18 rigide an dem Endeffektor 10 befestigt.

Das Navigationssystem 2 weist einen Patiententracker 20 zum Referenzieren der Position und Orientierung des Patienten auf. Der Patiententracker 20 ist eingerichtet, rigide an dem Patienten befestigt zu werden. Vorzugsweise ist der Patiententracker 20 direkt an dem Patienten oder indirekt über eine am Patienten befestigte Vorrichtung an dem Patienten befestigt.

Das Navigationssystem 2 weist eine Navigationskamera 22 auf. Vorzugsweise ist die Navigationskamera 22 stereoskopisch. Vorzugsweise ist die Navigationskamera 22 beweglich. Vorzugsweise ist die Navigationskamera 22 zur Nachverfolgung im OR-Spektrum (Infraroten Spektrum) oder im sichtbaren Spektrum eingerichtet. Die Navigationskamera 22 ist eingerichtet, den Robotertracker 18 (kontinuierlich) nachzuverfolgen und als Robotertracker-Positionsdaten Trob bereitzustellen. Dazu ist es erforderlich, dass sich der Robotertracker 18 innerhalb eines Sichtfelds der Navigationskamera 22 befindet. Die Navigationskamera 22 ist eingerichtet, den Patiententracker 20 (kontinuierlich) nachzuverfolgen und als Patiententracker-Positionsdaten Tpat bereitzustellen. Dazu ist es erforderlich, dass sich der Patiententracker 20 innerhalb des Sichtfelds der Navigationskamera 22 befindet.

Das Navigationssystem 2 weist eine Steuereinheit 24 auf.

Das Navigationssystem 2 weist ein Benutzereingabemittel 26 auf. Vorzugsweise ist das Benutzereingabemittel 26 in Form eines Touchdisplays/Touchpads ausgebildet. Vorzugsweise ist das Benutzereingabemittel 26 an dem Endeffektor 10 angebracht.

Das Navigationssystem 2 weist radiologische 3D-Aufnahmedaten 28 des Patienten auf.

Vorzugsweise kann das Navigationssystem 2 eine (nicht dargestellte) Aufnahmeeinheit aufweisen. Die Aufnahmeeinheit ist eingerichtet, eine zeitaktuelle Aufnahme des Patienten zu erstellen. Die zeitaktuelle Aufnahme kann eine bildbasierte Aufnahme des Patienten sein. Alternativ kann die zeitaktuelle Aufnahme eine pointerbasierte Aufnahme des Patienten sein. Die zeitaktuelle Aufnahme dient zur (initialen) Registrierung des Patienten.

Die Steuereinheit 24 ist eingerichtet, eine (initiale) Registrierung der radiologische 3D-Aufnahmedaten 28 zu dem Patienten, insbesondere anhand der zeitaktuellen Aufnahme des Patienten, durchzuführen und die radiologischen 3D-Aufnahmedaten 28 fest mit dem Patiententracker 20 zu verknüpfen.

Vorzugsweise kann das Navigationssystem 2 eine (nicht dargestellte) Patienten-Bewegungserfassungseinheit aufweisen. Die Patienten-Bewegungserfassungseinheit kann eingerichtet sein, eine Bewegung des Patienten zu erfassen.

Vorzugsweise kann das Navigationssystem 2 eine (nicht dargestellte) Roboterbasis-Bewegungserfassungseinheit, vorzugsweise in Form eines Sensors in einer Bremse der Roboterbasis, aufweisen. Die Roboterbasis-Bewegungserfassungseinheit kann eingerichtet sein, eine Bewegung der Roboterbasis 6 zu erfassen.

Vorzugsweise kann das Navigationssystem 2 eine (nicht dargestellte) Benutzereingabemittel-Bewegungserfassungseinheit, vorzugsweise in Form eines Sensors zur Drehmomentmessung in Gelenken des Roboterarms, aufweisen. Die Benutzereingabemittel-Bewegungserfassungseinheit kann eingerichtet sein, eine Bewegung des Benutzereingabemittels 26 zu erfassen.

Die Steuereinheit 24 ist eingerichtet, eine Transformationsmatrix auf Basis der Robotertracker-Positionsdaten Trob und der Patiententracker-Positionsdaten Tpat zu bestimmen. Die Steuereinheit 24 ist eingerichtet, eine (zeitliche) Veränderung Tchange der Transformationsmatrix Tref (kontinuierlich) zu überwachen. Die Überwachung dient insbesondere dazu, um eine Relativbewegung zwischen dem Patienten und dem Patiententracker 20 zu erkennen.

In einem roboterstatischen Fall (vgl. Fig. 1) kann die Steuereinheit 24 eingerichtet sein, eine erste Transformationsmatrix Tref1 als die Transformationsmatrix Tref zu verwenden. Dabei gibt die erste Transformationsmatrix Tref1 eine relative Position und eine relative Orientierung zwischen dem Robotertracker 18 und dem Patiententracker 20 an. Unter dem roboterstatischen Fall wird eine Situation verstanden, bei der sich der Roboterarm 8 während der Überwachung nicht bewegt hat. Dabei gilt: $Tchange = {Tpat}^{- 1} Trob Tref 1$

Die Steuereinheit 24 ist eingerichtet, Roboterbasis-Positionsdaten auf Basis der Robotertracker-Positionsdaten Trob und einer Roboterkinematik Tbas des Roboters 4 zu bestimmen.

In einem roboterdynamischen Fall (vgl. Fig. 2) kann die Steuereinheit 24 eingerichtet sein, eine zweite Transformationsmatrix Tref2 als die Transformationsmatrix Tref zu verwenden. Dabei gibt die zweite Transformationsmatrix Tref2 eine relative Position und eine relative Orientierung zwischen der Roboterbasis 6 und dem Patiententracker 20 an. Unter dem roboterdynamischen Fall wird eine Situation verstanden, bei der sich der Roboterarm 8 während der Überwachung bewegt hat. Dabei gilt: $Tchange = {Tpat}^{- 1} Trob {Tbas}^{- 1} Tref 2$

Vorzugsweise kann die Steuereinheit 24 eingerichtet sein, die Veränderung der Transformationsmatrix mit einem vorbestimmten Schwellenwert zu vergleichen. Insbesondere kann die Steuereinheit 24 eingerichtet sein, bei Überschreiten des vorbestimmten Schwellenwerts einen Hinweis für einen Benutzer auszugeben. Der Hinweis kann graphisch und/oder akustisch sein. Der Hinweis kann in Abhängigkeit der Veränderung der Transformationsmatrix variieren. Vorzugsweise kann die Steuereinheit 24 eingerichtet sein, bei Überschreiten des vorbestimmten Schwellenwerts einen Benutzer aufzufordern, eine Navigationsgenauigkeit manuell zu überprüfen

Vorzugsweise kann die Steuereinheit 24 eingerichtet sein, die (initiale) Registrierung, insbesondere (nur) in Reaktion auf eine Benutzereingabe, auf Basis der Veränderung Tchange der Transformationsmatrix Tref zu korrigieren. Insbesondere kann die Steuereinheit 24 eingerichtet sein, die Registrierung um einen Korrekturfaktor in Form einer Korrekturmatrix Tkor zu korrigieren.

Vorzugsweise kann die Steuereinheit 24 eingerichtet sein, die Veränderung Tchange der Transformationsmatrix Tref als die Korrekturmatrix Tkor zu verwenden. Dabei gilt: $Tkor = Tchange$

Insbesondere kann die Steuereinheit 24 eingerichtet sein, in dem roboterdynamischen Fall den Roboter 4 in eine vorbestimmte Position zu verfahren und dann die Veränderung Tchange der Transformationsmatrix Tref als die Korrekturmatrix Tkor zu verwenden.

Zudem kann die Steuereinheit 24 eingerichtet sein, nach Korrektur der Registrierung einen Benutzer aufzufordern, eine Navigationsgenauigkeit manuell zu überprüfen. Weiter kann die Steuereinheit 24 eingerichtet sein, auf Basis der Veränderung Tchange der Transformationsmatrix Tref einen Benutzer aufzufordern, die Registrierung zu wiederholen.

## Patentansprüche

1. Navigationssystem (2) für einen chirurgischen Eingriff an einem Patienten, mit einem Roboter (4), der eine Roboterbasis (6) und einen zu der Roboterbasis (6) beweglichen Roboterarm (8) mit einem Endeffektor (10) aufweist,
einem Robotertracker (18) zum Referenzieren der Position und Orientierung des Roboters (4), wobei der Robotertracker (18) rigide an dem Roboter (4) befestigt ist,
einem Patiententracker (20) zum Referenzieren der Position und Orientierung des Patienten, wobei der Patiententracker (20) eingerichtet ist, rigide an dem Patienten befestigt zu werden,
einer Navigationskamera (22), die eingerichtet ist, den Robotertracker (18) nachzuverfolgen und als Robotertracker-Positionsdaten (Trob) bereitzustellen sowie den Patiententracker (20) nachzuverfolgen und als Patiententracker-Positionsdaten (Tpat) bereitzustellen, und
einer Steuereinheit (24), die eingerichtet ist, eine Transformationsmatrix (Tref) auf Basis der Robotertracker-Positionsdaten (Trob) und der Patiententracker-Positionsdaten (Tpat) zu bestimmen und eine Veränderung (Tchange) der Transformationsmatrix (Tref) zu überwachen, um insbesondere eine Relativbewegung zwischen dem Patienten und dem Patiententracker (20) zu erkennen.

2. Navigationssystem (2) nach Anspruch 1, **dadurch gekennzeichnet, dass** die Steuereinheit (24) eingerichtet ist, die Veränderung (Tchange) der Transformationsmatrix (Tref) mit einem vorbestimmten Schwellenwert zu vergleichen und bei Überschreiten des vorbestimmten Schwellenwerts einen Hinweis für einen Benutzer auszugeben, wobei der Hinweis vorzugsweise graphisch und/oder akustisch ist.

3. Navigationssystem (2) nach Anspruch 1 oder 2, **dadurch gekennzeichnet, dass** die Steuereinheit (24) eingerichtet ist, die Veränderung (Tchange) der Transformationsmatrix (Tref) mit einem vorbestimmten Schwellenwert zu vergleichen und bei Überschreiten des vorbestimmten Schwellenwerts den Benutzer aufzufordern, eine Navigationsgenauigkeit manuell zu überprüfen.

4. Navigationssystem (2) nach einem der Ansprüche 1 bis 3, **dadurch gekennzeichnet, dass** der Robotertracker (18) rigide an dem Roboterarm (8), insbesondere dem Endeffektor (10), befestigt ist, wobei die Steuereinheit (24) eingerichtet ist, in einem roboterstatischen Fall eine erste Transformationsmatrix (Tref1) als die Transformationsmatrix (Tref) zu verwenden, wobei die erste Transformationsmatrix (Tref1) eine relative Position und eine relative Orientierung zwischen dem Roboterarm (8), insbesondere dem Endeffektor (10), und dem Patiententracker (20) angibt.

5. Navigationssystem (2) nach einem der Ansprüche 1 bis 4, **dadurch gekennzeichnet, dass** der Robotertracker (18) rigide an dem Roboterarm (8), insbesondere dem Endeffektor (10), befestigt ist und die Steuereinheit (24) eingerichtet ist, Roboterbasis-Positionsdaten auf Basis der Robotertracker-Positionsdaten (Trob) und einer Roboterkinematik (Tbas) des Roboters (4) zu bestimmen, wobei die Steuereinheit (24) eingerichtet ist, in einem roboterdynamischen Fall eine zweite Transformationsmatrix (Tref2) als die Transformationsmatrix (Tref) zu verwenden, wobei die zweite Transformationsmatrix (Tref2) eine relative Position und eine relative Orientierung zwischen der Roboterbasis (6) und dem Patiententracker (20) angibt.

6. Navigationssystem (2) nach Anspruch 4 und 5, **dadurch gekennzeichnet, dass** die Steuereinheit (24) eingerichtet ist, in dem roboterstatischen Fall einen ersten vorbestimmten Schwellenwert und in dem roboterdynamischen Fall einen zweiten vorbestimmten Schwellenwert als den Schwellenwert zu verwenden, wobei der erste vorbestimmte Schwellenwert und der zweite vorbestimmte Schwellenwert gleich sind oder unterschiedlich zueinander sind.

7. Navigationssystem (2) nach einem der Ansprüche 1 bis 6, **dadurch gekennzeichnet, dass** das Navigationssystem (2) radiologische 3D-Aufnahmedaten (28) des Patienten aufweist, und die Steuereinheit (24) eingerichtet ist, eine Registrierung der radiologische 3D-Aufnahmedaten (28) zu dem Patienten durchzuführen und die radiologischen 3D-Aufnahmedaten (28) fest mit dem Patiententracker (20) zu verknüpfen.

8. Navigationssystem (2) nach Anspruch 7, **dadurch gekennzeichnet, dass** die Steuereinheit (24) eingerichtet ist, die Registrierung, insbesondere in Folge einer Benutzereingabe, auf Basis der Veränderung (Tchange) der Transformationsmatrix (Tref) um einen Korrekturfaktor in Form einer Korrekturmatrix (Tkor) zu korrigieren.

9. Navigationssystem (2) nach Anspruch 8, **dadurch gekennzeichnet, dass** die Steuereinheit (24) eingerichtet ist, die Veränderung (Tchange) der Transformationsmatrix (Tref) als die Korrekturmatrix (Tkor) zu verwenden, wobei die Steuereinheit (24) eingerichtet ist, in dem roboterdynamischen Fall den Roboter in eine vorbestimmte Position zu verfahren und dann die Veränderung (Tchange) der Transformationsmatrix (Tref) als die Korrekturmatrix (Tkor) zu verwenden.

10. Navigationssystem (2) nach Anspruch 8 oder 9, **dadurch gekennzeichnet, dass** die Steuereinheit (24) eingerichtet ist, nach Korrektur der Registrierung einen Benutzer aufzufordern, eine Navigationsgenauigkeit manuell zu überprüfen und vorzugsweise anschließend zu bestätigen.

11. Navigationssystem (2) nach einem der Ansprüche 7 bis 10, **dadurch gekennzeichnet, dass** die Steuereinheit (24) eingerichtet ist, auf Basis der Veränderung (Tchange) der Transformationsmatrix (Tref) einen Benutzer aufzufordern, die Registrierung zu wiederholen.

12. Navigationssystem (2) nach einem der Ansprüche 1 bis 11, **dadurch gekennzeichnet, dass** das Navigationssystem (2) eine Patienten-Bewegungserfassungseinheit aufweist, die eingerichtet ist, eine Bewegung des Patienten zu erfassen, und die Steuereinheit (24) eingerichtet ist, die Transformationsmatrix (Tref), insbesondere in Folge einer Benutzereingabe, auf Basis eines Vergleichs der Bewegung des Patienten und der Veränderung (Tchange) der Transformationsmatrix (Tref) zu korrigieren.
